# EUROPEAN PATENT APPLICATION

(11) **EP 4 108 766 A1**
(43) Date of publication of application: **28.12.2022**
(21) Application number: 21757937.4
(22) Date of filing: 15.02.2021
(51) Int. Cl.: C12N 5/09, C12N 5/071, C12M 3/00

(54) **METHOD FOR PREPARING FIBROSIS-ENCAPSULATED TUMOROID, AND USE THEREOF**

(30) Priority: 18.02.2020 KR 20200019681
(71) Applicant: Industry-Academic Cooperation Foundation, Yonsei University, Seoul 03722 (KR)
(72) Inventor: KIM, Baek Gil, Seoul 04305 (KR); JANG, Yeonsue, Seoul 04305 (KR); CHO, Nam Hoon, Seoul 06098 (KR); KANG, Suki, Paju-si Gyeonggi-do 10916 (KR)
(74) Representative: Schnappauf, Georg
(86) International application number: PCT/KR2021/001865
(87) International publication number: WO 2021/167297

(57) **Abstract**

The present invention relates to a method for producing a fibrosis-encapsulated tumoroid (FET), and the use of the fibrosis-encapsulated tumoroid. According to the present invention, an analogue that is close to real solid cancer tissue is produced using induced pluripotent stem cell-derived cell. The analogue has significant improvement over conventional tumoroids, which fail to perfectly reflect the characteristics of human solid cancer and have a very high probability of failure in the clinical validation stage. Thus, the present invention is expected to be widely used in the fields of new anticancer drug development and precision medicine.

## Description

### Technical Field

The present invention relates to a method for producing a fibrosis-encapsulated tumoroid model that mimics the process of drug delivery to human solid cancer, and a method of screening a substance for treatment or alleviation of cancer by using the same.

### Background Art

Cancer is one of the incurable diseases to be resolved by mankind, and huge capital has been invested in development to cure cancer worldwide. In Korea, cancer is the number one cause of death by disease, and more than 100,000 people are diagnosed with cancer annually, and more than 60,000 people die from cancer. In the past decade, there have been rapid developments in cancer diagnosis and treatment, but the mortality rate due to cancer is still high.

Existing anticancer drugs developed through new drug development research are not actively utilized for actual cancer patients because they show side effects at the clinical stage or have low efficacy. In addition, even patients with the same cancer have different responsivenesses to the same anticancer drug in many cases, and thus research thereon is urgently needed. In recent years, for this new drug development research and as various cancer cell spheroids for different individuals, a number of tumoroids that are tumor-like organoids have been reported. When the tumoroids are composed of various cells, they are referred to as heterocellular or multi-cellular tumoroids. However, the currently widely used screening methods based on two-dimensional culture or simple spheroids do not fully reflect the characteristics of human solid cancer, and thus there is a problem that drugs developed through the screening methods are highly likely to fail to pass through the clinical trial stage.

A new tumoroid culture method that can satisfy this demand is a three-dimensional fibrosis-encapsulated tumoroid culture method on which the present invention is based. Common existing organoids are spheroids made using stem cells or cancer tissue-derived cells, and show differentiation similar to that of organs or cancer tissues (Sci Rep. 2016 Jan 11;6:19103.). However, it cannot be considered that these organoids perfectly mimic actual cancer tissue. A fibrotic tissue structure surrounding cancerous tissue is a characteristic shared by all solid cancers, and cannot be formed naturally by an existing organoid culture method and must be formed artificially. Therefore, there is a need for development of a method of artificially forming a fibrotic layer on a tumoroid so as to perfectly reflect the fibroblast structure and vascular structure appearing in actual human solid cancer and to mimic that in the human body.

The present inventors have invented a fibrosis-encapsulated tumoroid model that reflects the characteristics of human solid cancer and furthermore, can be widely used in new drug development by mimicking the process of drug delivery from blood vessels to cancer cells, that is, the same drug delivery process as in the human body. In addition, when the fibrosis-encapsulated tumoroid model is used, a more optimal method than an existing method may be applied, so that the cost and time can be reduced, and thus it is possible to preemptively select anticancer drugs for cancer patients who have time and physical limitations. Therefore, the fibrosis-encapsulated tumoroid model is expected to become a stepping stone for providing customized medical care to actual patients.

### DISCLOSURE

### Technical Problem

An object of the present invention is to provide a method for culturing a three-dimensional fibrosis-encapsulated tumoroid composed of a fibrotic layer and/or vascular cell layer having the same characteristics as that of human solid cancer tissue with high yield in a short time.

Another object of the present invention is to provide a fibrosis-encapsulated tumoroid produced according to the method of the present invention.

Still another object of the present invention is to provide a non-human animal model transplanted with a fibrosis-encapsulated tumoroid produced according to the method of the present invention.

Yet another object of the present invention is to provide a method of screening a substance for treatment or alleviation of cancer by using a fibrosis-encapsulated tumoroid provided in the present invention.

However, objects to be achieved by the present invention are not limited to the objects mentioned above, and other objects not mentioned herein will be clearly understood by those of ordinary skill in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present invention pertains.

The present invention is directed to a method for producing a fibrosis-encapsulated tumoroid (Fibrosis-encapsulated tumoroid, FET) and various uses of the fibrosis-encapsulated tumoroid produced thereby.

According to one embodiment of the present invention, the present invention is directed to a method for producing a fibrosis-encapsulated tumoroid.

In one embodiment of the present invention, the term "tumoroid" refers to a cell aggregate having cancer cell characteristics, which is a kind of organoid that is a cell aggregate made by aggregation or recombination of cells isolated from stem cells or cells originated in organ by 3D culture method. This term may cover a tumoroid or cell cluster formed from a suspension cell culture. In the present specification, the term "tumoroid" may be used interchangeably with the term "solid cancer analogue", "organoid", or "spheroid".

In one embodiment of the present invention, the "organoid" is a spheroid made of stem cells or cancer tissue-derived cells and shows differentiation similar to organs or cancer tissues. The organoid specifically includes one or more cell types among various types of cells constituting an organ or tissue, and should be able to reproduce the shape and function of the tissue or organ. When the organoid is produced using cells from a patient with a specific disease, it can provide conditions that perfectly reflect genetic, physiological and environmental characteristics. Thus, the organoid is widely used in drug screening to select suitable drugs for each individual.

The method for producing a fibrosis-encapsulated tumoroid according to the present invention may comprise a step of obtaining cancer cells isolated from a subject.

As used herein, the term "cancer tissue" and "cancer cells" are used in the same sense as the terms "tumor tissue" and "tumor cells", respectively.

In the present invention, the cancer cells are cells harvested from cancer patients, and the "cancer" refers to or describes the physiological condition in mammals that is typically characterized by unregulated cell growth. In the present invention, the cancer may be a cancer selected from the group consisting of breast cancer, uterine cancer, esophageal cancer, stomach cancer, brain cancer, rectal cancer, colorectal cancer, lung cancer, skin cancer, ovarian cancer, cervical cancer, kidney cancer, blood cancer, pancreatic cancer, prostate cancer, testicular cancer, laryngeal cancer, oral cancer, head and neck cancer, thyroid cancer, liver cancer, bladder cancer, osteosarcoma, lymphoma, leukemia, and combinations thereof, depending on the site of development thereof, but is not limited thereto.

In the present invention, the cancer cells generally correspond to spheroid-forming cells, but the spheroid-forming ability as described above is not applicable to all types of cancer cells, and some types of cancer cells have the characteristics of non-spheroid-forming cells (NSFs).

The method for producing a fibrosis-encapsulated tumoroid according to the present invention may comprise a step of co-culturing induced pluripotent stem cells (iPSCs) or induced pluripotent stem cell-derived cell together with the cancer cells to form a spheroid-shaped culture.

In one embodiment of the present invention, the term "induced pluripotent stem cell (iPSCs)" is a type of stem cells having the ability to differentiate into two or more cell types while having self-replicating ability. Stem cells with pluripotency, such as embryonic stem cells, may be produced by introducing and expressing four specific dedifferentiation-inducing genes in somatic cells such as adult skin cells having no pluripotency, or extracting a dedifferentiation-inducing protein produced in cells into which four dedifferentiation-inducing genes have been introduced and injecting the dedifferentiation-inducing protein into somatic cells. These stem cells are called induced pluripotent stem cells or dedifferentiated stem cells. In 2006, pluripotent stem cells like embryonic stem cells were successfully created by introducing several genes into mouse skin fibroblasts, and in 2007, induced pluripotent stem cells were successfully created by introducing genes into adult skin cells. These induced pluripotent stem cells are created by dedifferentiating already mature and differentiated cells into immature cells that can develop into any tissue, and studies using these induced pluripotent stem cells are currently actively conducted in various fields.

In the present invention, the cancer cells may include non-spheroid-forming cells (NSFs), and NSFs may be formed into spheroids by co-culture with induced pluripotent stem cells or cells derived therefrom, preferably induced pluripotent stem cell-derived mesenchymal stem cells (iPSC-derived MSCs, iMSCs), which are linker cells.

In the present invention, for co-culture of the cancer cells with the induced pluripotent stem cells or induced pluripotent stem cell-derived cells, these cells may be mixed together and co-cultured at a cell number ratio of 0.01:1 to 100:1, preferably 0.1:1 to 10:1, 1:1 to 5:1, or 1.5:1 to 2.5:1.

In the present invention, a medium composition that is used in co-culture of the cancer cells with the induced pluripotent stem cells or induced pluripotent stem cell-derived cells may contain the induced pluripotent stem cells or induced pluripotent stem cell-derived cells in an amount of 1 × 10⁴ cells/cm² to 10 × 10⁴ cells/cm², 2 × 10⁴ cells/cm² to 8 × 10⁴ cells/cm², 3 × 10⁴ cells/cm² to 7 × 10⁴ cells/cm², or 4 × 10⁴ cells/cm² to 6 × 10⁴ cells/cm², without being limited thereto.

In addition, in the present invention, the culture medium that is used in the co-culture may be, for example, at least one culture medium selected from the group consisting of DMEM (Dulbeco's modified Eagle's medium), IMDM (Iscove's modified Dulbecco's medium), a-MEM (alpha modification of Eagle's medium), TI-free OGM (TI-free osteoblast growth medium), F12 (nutrient mixture F-12), RPMI 1640 (RPMI 1640 medium), Williams' s medium E, McCoy's 5A, essential 8 (E8) medium, SFM medium (StemPro-34 SFM medium), N2B2 medium, OBM medium (OGM osteoblast growth medium), growth medium-5, growth medium-10, and DMEM/F12 (Dulbecco's modified Eagle medium: nutrient mixture F-12), without being particularly limited thereto. In addition, the medium may further contain at least one of B27 supplement, N2 supplement, G5 supplement, or forskolin.

In addition, in the present invention, the culture medium that is used in the co-culture may further contain at least one of cancer-associated fibroblasts; endothelial cells; mesenchymal stem cells (iMSCs) differentiated from induced pluripotent stem cells (iPSCs); and a growth factor.

In the present invention, the growth factor may be at least one selected from the group consisting of vascular endothelial growth factor (VEGF), vascular endothelial growth factor A (VEGFA), transforming growth factor beta (TGF-β), basic fibroblast growth factor (bFGF), epidermal growth factor (EGF), and insulin-like growth factor-1 (IGF-1), and may preferably be transforming growth factor beta, without being limited thereto.

The method for producing a fibrosis-encapsulated tumoroid according to the present invention may comprise a step of inducing a fibrotic layer in the culture formed as described above.

In one embodiment of the present invention, the term "fibroblasts" refers to native cells of connective tissue, which have an ellipsoidal nucleus and fusiform protoplast and are characterized by good development of the rough endoplasmic reticulum and the Golgi body. The fibroblasts are closely related to myoblasts (myoplast), and in primary culture of cancer cells or muscle, etc., the term "fibroblasts" collectively refers to existing cells other than parenchymal cells. The fibroblasts proliferate by serum or fibroblast growth factor (FGF). The fibroblasts are used for cancer screening using the loss of function for contact inhibition, which is characteristic of cancer cells. Furthermore, the fibroblasts are frequently used for cell proliferation studies, in particular, studies on the signaling mechanisms of growth factors (growth factor).

In one embodiment of the present invention, the term "endothelial cells" refers to a single layer of squamous cells covering the inner walls of blood vessels and lymphatic vessels. Most of epithelial organs are originally ectodermal or endodermal, and the walls of the serous cavity or the inner walls of blood vessels and lymphatic vessels, which are produced from the mesenchyme, were previously called pseudoepithelium, but are now termed endothelium. It is known that, in single-layer squamous epithelium, thin plate-like cells are arranged in one layer and tightly coupled to one another on the outer surfaces of the cells. Usually, endothelial cells form the lining of the inner surface of all blood vessels and constitute the non-thrombogenic interface between blood and tissue. Endothelial cells are also important components for the development of new capillaries and blood vessels, and endothelial cells proliferate during new blood vessel formation or neovascularization processes associated with tumor growth and metastasis as well as various non-neoplastic diseases or disorders.

In one embodiment of the present invention, the term "vascular endothelial cells" refers to a single layer of cells constituting the inner membrane by the lining of blood vessels. The vascular endothelial cells are of mesodermal origin. Since it was suggested that the vascular relaxation phenomenon caused by acetylcholine is lost due to intimal dissection, the functional significance of the vascular endothelial cells has attracted attention. It is known that, in addition to vascular tone control by production of endothelial cell-derived smooth muscle relaxation factor, which mediates such relaxation, or endothelin which acts as a vasoconstrictor, the vascular endothelial cells have various functions, including regulation of intravascular blood coagulation and vascular permeability, substance exchange, and biological defense by interaction with leukocytes.

As used herein, the term "vascular endothelial cell growth factor" refers to a polypeptide which is produced in cells, stimulates new blood vessel formation, and is isolated from the supernatant of the cultured pituitary cells using the growth activity for vascular endothelial cells as an indicator. It is known that the vascular endothelial cell growth factor is produced as a yeast two-hybrid by S-S bonding through of translation of four polypeptides having 121, 165, 189, and 206 amino acid residues, respectively, from single genes, and is produced by various cells, including macrophages, smooth muscle cells, tumor cells, etc.

In the present invention, the "transforming growth factor beta" refers to a protein having a 25 kDa dimeric structure, which binds to a TGF-β receptor (transforming growth factor beta receptor, TGF-β receptor) that transmits a signal into the cell, thus activating serine/threonine kinase and transmitting a signal into the cell. There are three types of isoforms (TGF-β1, β2 and β3) having similar structures in mammals. Transforming growth factor beta was originally discovered as one of the substances that promote proliferation of fibroblasts in soft agar, but is now known as a growth inhibitory factor for most cells. It is known that TGF-β is produced in various tissues such as platelets, placenta, and skeleton, and acts to inhibit cell growth, produce extracellular matrix, and suppress immunity.

In the present invention, the fibrotic layer may be induced by at least one cell type selected from the group consisting of fibroblasts, fibroblast precursor cells, cancer-associated fibroblasts, induced pluripotent stem cells-derived mesenchymal stem cells (iMSCs), and iPSC-derived cancer-associated fibroblasts, and but is not limited thereto and may be induced by any cell type having characteristics similar to those of fibroblasts.

In order to induce the fibroblast layer of the present invention, the culture and the induced pluripotent stem cells or induced pluripotent stem cell-derived cells may be mixed together and co-cultured to induce the fibroblast layer on the outside of the culture.

In the present invention, the induced pluripotent stem cell-derived cells may be at least one cell type selected from among induced pluripotent stem cells-derived mesenchymal stem cells (iMSCs), and iPSC-derived cancer-associated fibroblasts.

In the present invention, the induced pluripotent stem cells or induced pluripotent stem cell-derived cells may interact with the cancer cells present in the culture and induced to differentiate into cancer-associated fibroblasts by TGF-β secreted by the cancer cells.

In the present invention, the induced pluripotent stem cells or induced pluripotent stem cell-derived cells and the culture may be mixed together and co-cultured at a cell number ratio of 5:1 to 2:1, preferably 3:1.

A medium composition that is used in co-culture of the induced pluripotent stem cells or induced pluripotent stem cell-derived cells and the culture may contain the induced pluripotent stem cells or induced pluripotent stem cell-derived cells in an amount of 1 × 10⁴ cells/cm² to 10 × 10⁴ cells/cm², 2 × 10⁴ cells/cm² to 8 × 10⁴ cells/cm², 3 × 10⁴ cells/cm² to 7 × 10⁴ cells/cm², or 4 × 10⁴ cells/cm² to 6 × 10⁴ cells/cm².

In the present invention, the medium composition that is used in co-culture of the induced pluripotent stem cells or induced pluripotent stem cell-derived cells and the culture may further contain a growth factor. Here, the growth factor may be at least one selected from the group consisting of vascular endothelial growth factor (VEGF), vascular endothelial growth factor A (VEGFA), transforming growth factor beta (TGF-β), basic fibroblast growth factor (bFGF), epidermal growth factor (EGF), and insulin-like growth factor-1 (IGF-1), and may preferably be transforming growth factor beta, but is not limited thereto.

In addition, the amount of the growth factor further contained in the medium composition that is used in co-culture of the induced pluripotent stem cells or induced pluripotent stem cell-derived cells and the culture may be 10 to 1000 ng/ml, 50 to 500 ng/ml, 60 to 200 ng/ml, or 80 to 120 ng/ml, but is not limited thereto.

The method for producing a fibrosis-encapsulated tumoroid according to the present invention may, if necessary, further comprise a step of compacting the fibrotic layer induced as described above.

In the present invention, compaction of the fibrotic layer may be performed by further culturing at a temperature of 35 to 40°C under 5 volume% CO₂ for 1 to 10 days, preferably 1 to 5 days.

The method for producing a fibrosis-encapsulated tumoroid according to the present invention may comprise a step of selecting a fibrosis-encapsulated tumoroid (FET) on which the fibrotic layer has been induced.

In addition, the method for producing a fibrosis-encapsulated tumoroid according to the present invention may comprise a step of inducing a vascular cell layer on the selected spheroid (FET).

In order to induce the vascular cell layer of the present invention, the FET and endothelial cells or vascular endothelial cells may be mixed together and co-cultured to induce the vascular cell layer on the outside of the FET.

In the present invention, the endothelial cells or vascular endothelial cells may be cells induced by differentiation from induced pluripotent stem cells, preferably cells formed from endothelial cells or vascular endothelial cells derived from induced pluripotent stem cells-derived mesodermal cells, but are not limited thereto and may include any cell type having characteristics similar to those of the above cells.

In the present invention, the endothelial cells or vascular endothelial cells and the FET may be mixed together and co-cultured at a cell number ratio of 5:1 to 2:1, preferably 3:1.

In the present invention, a medium composition that is used in co-culture of the FET and the endothelial cells or vascular endothelial cells may contain the induced pluripotent stem cells or induced pluripotent stem cell-derived cells in an amount of 1 × 10⁴ cells/cm² to 10 × 10⁴ cells/cm², 2 × 10⁴ cells/cm² to 8 × 10⁴ cells/cm², 3 × 10⁴ cells/cm² to 7 × 10⁴ cells/cm², or 4 × 10⁴ cells/cm² to 6 × 10⁴ cells/cm².

In addition, in the present invention, the medium composition that is used in co-culture of the FET and the endothelial cells or vascular endothelial cells may further contain a growth factor. Here, the growth factor may be at least one selected from the group consisting of vascular endothelial growth factor (VEGF), vascular endothelial growth factor A (VEGFA), transforming growth factor beta (TGF-β), basic fibroblast growth factor (bFGF), epidermal growth factor (EGF), and insulin-like growth factor-1 (IGF-1), and may preferably be transforming growth factor beta, without being limited thereto.

In addition, in the present invention, the amount of the growth factor further contained in the medium composition that is used in co-culture of the FET and the endothelial cells or vascular endothelial cells may be 10 to 1,000 ng/ml, 50 to 500 ng/ml, 60 to 200 ng/ml, or 80 to 120 ng/ml, but is not limited thereto.

In the present invention, co-culture of the FET and the endothelial cells or vascular endothelial cells may be performed for 2 days to 14 days, or 3 days to 10 days, preferably 4 days to 7 days, without being limited thereto.

In the present invention, co-culture of the FET and the endothelial cells or vascular endothelial cells may be performed at a temperature of 37°C under 5% CO₂, without being limited thereto.

The method for producing a fibrosis-encapsulated tumoroid according to the present invention may, if necessary, further comprise a step of compacting the vascular cell layer induced as described above.

The method for producing a fibrosis-encapsulated tumoroid according to the present invention may comprise a step of selecting a spheroid (endothelial cell-layered FET (eFET) on which the vascular cell layer has been induced.

According to another embodiment of the present invention, the present invention is directed to a fibrosis-encapsulated tumoroid produced by the method of the present invention.

The fibrosis-encapsulated tumoroid according to the present invention is obtained by three-dimensional culture of cancer cells isolated from a cancer patient, and is characterized by being obtained by compacting the fibrotic layer on the outer layer of the tumoroid, which is a structural characteristic of solid cancer in the human body, through the encapsulation process. Furthermore, in the present invention, formation of the additional vascular cell layer on the tumoroid encapsulated with the fibrotic layer is induced, and thus a tumoroid screened after treating the final produced tumoroid with a drug has a technological characteristic in that it can mimic a drug delivery process appearing in human solid cancer tissue, that is, a series of processes in which an anticancer drug passes sequentially through the blood vessel wall and fibrotic tissue and reaches cancer tissue, in the same manner as those in the human body.

In general, cancer cells are composed of cells having spheroid-forming ability and cells having non-spheroid-forming ability. It is considered that the present invention differs from conventional inventions in that it is possible to widen the types of cancer capable of producing tumoroids because it is possible to form spheroids from even cancer cells having non-spheroid-forming ability by using pluripotent stem cells.

In addition, in the present invention, patient-derived cancer cells may be used to store the fibrosis-encapsulated tumoroid or to mass-produce the fibrosis-encapsulated tumoroid by continuous culture of the cancer cells.

According to still another embodiment of the present invention, the present invention is directed to a non-human animal model transplanted with the fibrosis-encapsulated tumoroid produced by the method of the present invention.

As used herein, the term "animal model" refers to a disease animal model. Specifically, the animal model may be an animal model afflicted with a disease similar to a human disease or an animal model created so as to be congenitally afflicted with the disease. In the present specification, the animal model may be an animal model of allergic respiratory disease. In addition, in the present invention, animals that may be used as the animal model of allergic respiratory disease may be mammals other than humans, and may be, for example, selected form the group consisting of rats, mice, guinea pigs, hamsters, rabbits, monkeys, dogs, cats, cattle, horses, pigs, sheep and goats. More preferably, the animals may be mice.

According to yet another embodiment of the present invention, the present invention is directed to a method of screening an anticancer agent for treatment or alleviation of cancer by using a fibrosis-encapsulated tumoroid produced by the method of the present invention.

As used herein, the term "anticancer agent" or "anticancer drug" refers to a candidate substance screened using the fibrosis-encapsulated tumoroid of the present invention. Examples of the anticancer agent include, without limitation, any agents that inhibit or delay the proliferation of cancer cells or cancer stem cells. Specific examples of the anticancer agent include anti-cancer chemotherapeutic agents, targeted anti-cancer agents, immunotherapeutic anti-cancer agents, metabolic anti-cancer agents, etc., and also include, without limitation, any type of drug that attacks rapidly dividing cancer cells or cancer stem cells after administration *in vivo.*

In one embodiment of the present invention, the term "screening" refers to one of a number of procedures that are performed during new drug development, and more specifically, refers to a procedure of determining a candidate substance after identifying active substances and lead substances through screening of compounds selected from basic research. The screening can reduce the time and cost required to develop a new drug, and after a target disease is selected, it is possible to identify a new effective drug substance targeting the disease. Examples of this substance include, without limitation, not only new drugs but also any substance that alleviates or beneficially changes symptoms of a skin disease, more specifically atopic dermatitis disease, when administered as a preventive agent or a cosmetic composition.

The method for screening an anticancer agent according to the present invention may comprise a step of preparing the fibrosis-encapsulated tumoroid of the present invention.

In the present invention, the fibrosis-encapsulated tumoroid exhibits the characteristics of human solid cancer and at the same time, perfectly mimics the drug delivery process appearing in human solid cancer, and thus may be used for screening a patient-specific therapeutic agent.

In the present invention, the method for screening an anticancer agent may comprise a step of treating the fibrosis-encapsulated tumoroid with a candidate substance.

In the present invention, the candidate substance may be at least one selected from the group consisting of natural compounds, synthetic compounds, RNA, DNA, polypeptides, enzymes, proteins, ligands, antibodies, antigens, bacterial or fungal metabolites, and bioactive molecules, without being limited thereto.

In addition, in the present invention, the candidate substance includes a drug having a mechanism to kill cancer cells. In a specific embodiment, the drug may comprise at least one selected from the group consisting of nitrogen mustard, imatinib, oxaliplatin, rituximab, erlotinib, neratinib, lapatinib, gefitinib, vandetanib, nilotinib, semaxanib, bosutinib, axitinib, cediranib, lestaurtinib, trastuzumab, gefitinib, bortezomib, sunitinib, carboplatin, bevacizumab, cisplatin, cetuximab, Viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate, chitosan, gemcitabine, doxifluridine, pemetrexed, tegafur, capecitabine, gimeracil, oteracil, azacitidine, methotrexate, uracil, cytarabine, fluorouracil, fludarabine, enocitabine, flutamide, decitabine, mercaptopurine, thioguanine, cladribine, carmofur, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, peplomycin, temsirolimus, temozolomide, busulfan, ifosfamide, cyclophosphamide, melphalan, altretamine, dacarbazine, thiotepa, nimustine, chlorambucil, mitolactol, leucovorin, tretinoin, exemestane, aminogluthetimide, anagrelide, navelbine, fadrozole, tamoxifen, toremifene, testolactone, anastrozole, letrozole, vorozole, bicalutamide, lomustine, and carmustine, but is not limited thereto.

The method for screening an anticancer agent according to the present invention may comprise a step of determining that the candidate substance is a substance for treatment or alleviation of cancer, when the fibrosis-encapsulated tumoroid treated with the candidate substance exhibits an anticancer effect.

Since the method for screening an anticancer agent according to the present invention uses a fibrosis-encapsulated tumoroid produced by perfectly reflecting the characteristics of solid cancer in the human body, provided by the present invention, it enables the preemptive selection of an optimal anticancer agent for each individual, and thus the method is expected to be widely used in the development of patient-specific anticancer therapeutic agents.

### Advantageous Effects

The present invention relates to a method for producing a fibrosis-encapsulated tumoroid exhibiting the characteristics of human solid cancer, and the use of the fibrosis-encapsulated tumoroid. The fibrosis-encapsulated tumoroid provided in the present invention perfectly reflects the structural characteristics of human solid cancer because the fibrotic layer and the vascular cell layer are formed on the outer layer of the tumoroid through the encapsulation process. When the fibrosis-encapsulated tumoroid is treated with a drug, it may perfectly mimic an *in vivo* drug delivery process, that is, a series of processes in which the drug passes sequentially through the blood vessel wall and fibrotic tissue and reaches cancer tissue. Therefore, when the fibrosis-encapsulated tumoroid according to the present invention is used, it is possible to increase the success rate in the clinical validation stage through accurate drug screening and preemptively select an optimal anticancer agent for each individual in a more effective manner by using patient-derived cancer cells. Therefore, the fibrosis-encapsulated tumoroid is expected to be widely used in the development of patient-specific anticancer therapeutic agents.

### Brief Description of Drawings

FIG. 1 is a schematic view showing an experimental design for producing a solid cancer analogue using induced pluripotent stem cells.

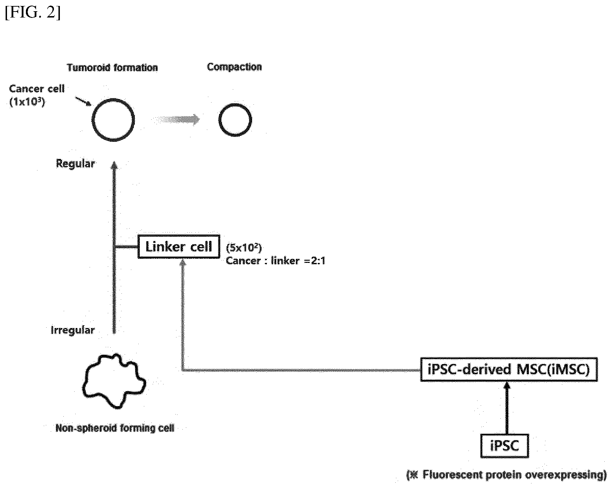

FIG. 2 shows a process of inducing spheroid formation from non-spheroid-forming cells (NSCs).
FIG. 3 shows results indicating that spheroid formation was induced from various cancer cells.
FIG. 4 shows results indicating the natural death of linker cells.
FIG. 5 is a schematic view sequentially showing processes of inducing of FET and eFET, which are fibrosis-encapsulated tumoroids.
FIG. 6 shows a series of processes of screening an anticancer agent using the fibrosis-encapsulated tumoroid.
FIG. 7 shows the results of observing the morphology of specific fibroblasts shown in various breast cancer cell lines.
FIGS. 8a and 8b show images after fluorescence staining of a fibrosis-encapsulated tumoroid (FET or eFET).
FIG. 9 shows the results of confirming the possibility of controlling the thickness of a fibrotic layer using fibroblasts.
FIGS. 10a and 10b show the results of comparing anticancer agent resistance between a simple tumoroid and a fibrosis-encapsulated tumoroid.

### Best Mode

One embodiment of the present invention is directed to a method for producing a fibrosis-encapsulated tumoroid, the method comprising steps of: (a) obtaining cancer cells isolated from a subject; and (b) co-culturing the cancer cells with induced pluripotent stem cells (iPSCs) or induced pluripotent stem cell-derived cells to form a spheroid-shaped culture.

Another embodiment of the present invention is directed to a fibrosis-encapsulated tumoroid produced by the method of the present invention.

Still another embodiment of the present invention is directed to a non-human animal model transplanted with the fibrosis-encapsulated tumoroid produced by the method of the present invention.

Yet another embodiment of the present invention is directed to a method of screening an anticancer agent using the fibrosis-encapsulated tumoroid produced by the method of the present invention.

### Mode for Invention

Hereinafter, the present invention will be described in more detail with reference to examples. These examples are only for illustrating the present invention in more detail, and it will be apparent to those of ordinary skill in the art that the scope of the present invention according to the subject matter of the present invention is not limited by these examples.

### Example 1: Culture of cancer cell lines and preparation of linker cells

### 1.1 Culture of cancer cell lines

The present inventors conducted all experiments with the approval of the Institutional Review Board (IRB) of the Yonsei University College of Medicine, and used the previously established induced pluripotent stem cell pool in the experiments. In addition, human breast cancer cell lines (BT-474, MCF-7, SK-BR-3, BT-20, MDA-MB-231 and Hs578T) and pancreatic cancer cell lines (PANC-1, MIA Paca-2, AsPC-1 and BxPC-3) were obtained from the American Type Culture Collection (ATCC), cultured in 10% FBS-containing media, and used in the experiment. All media used in the Examples below to culture other cells in addition to the culture of cancer cells have known compositions or are commercially available products. In the present invention, for optimization of culture, a mixture of two or more culture media was used or VEGFA or the like was added to the medium.

### 1.2 Preparation of linker cells

Using an embryonic body (EB) formation medium (EBFM, AggreWell, STEMCELL Technologies), an embryonic body (EB) was prepared with 1×10⁴ induced pluripotent stem cells (iPSCs). The embryo body was cultured for 5 days in Essential 8 (E8) medium containing the components shown in Table 1 below. On day 5, the embryo body was attached to a 0.1% gelatin coated dish and additionally cultured for 2 weeks in a mesenchymal stem cell (MSC) culture medium composed of the components shown in Table 2 below and allowed to differentiate into iPSC-derived mesenchymal cells (iMSCs), thus preparing linker cells.

**[Table 1]**

| **Essential 8 (E8) medium component** | **Final concentration** |
|---|---|
| DMEM/F12 with glutamine and HEPES | 1 x |
| NaHCO₃ | 543 µg/ml |
| Sodium selenite | 14 ng/ml |
| Transferrin | 10.7 µg/ml |
| Insulin | 19.4 µg/ml |
| L-ascorbic acid-2-phosphate magnesium | 64 µg/ml |
| FGF2 | 100 ng/ml |
| TGF-b1 | 100 ng/ml |

**[Table 2]**

| **MSC culture medium component** | **Final concentration** |
|---|---|
| Minimum Essential Medium Eagle - alpha modified (a-MEM) | 1 x |
| Fetal calf serum (FCS) | 10% |
| penicillin/streptomycin | 50 U·ml⁻¹ / 50 mg·ml⁻¹ |
| Sodium pyruvate | 1 mM |
| 1-ascorbate-2-phosphate | 100 µM |
| 1-Glutamine | 2 mM |
| non-essential amino acids | 1 x |
| HEPES | 10 mM |

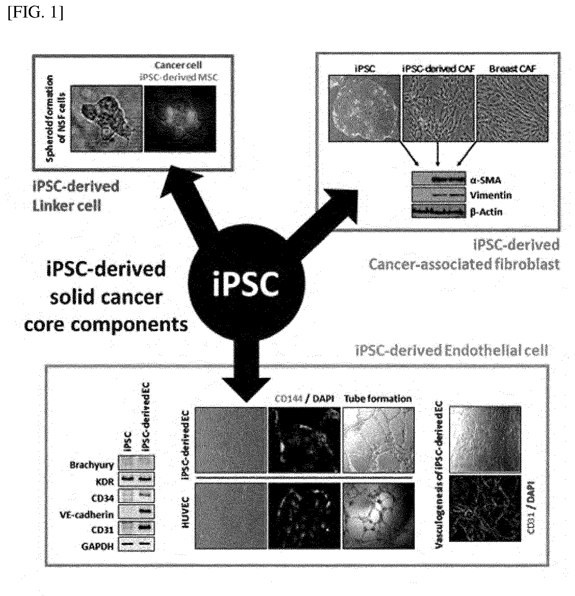

The present invention utilized induced pluripotent cells in a manner differentiated from conventional inventions, and FIG. 1 is a schematic view showing an experimental design for producing a solid cancer analogue using the induced pluripotent cells. Hereinafter, a process of producing a fibrosis-encapsulated tumoroid according to the design will be described in detail.

### Example 2: Induction of tumoroid formation from non-spheroid-forming cancer cells

Cancer cells and iMSCs were detached from culture plates and washed with PBS, and then a pellet form was prepared. Cancer cells (8 × 10⁴ cells/ml) and iMSCs (4 × 10⁴ cells/ ml) were prepared using a mixed medium of organoid basal medium (OBM) and EB formation medium (EBFM). Here, the mixed medium was maintained at an OBM: EBFM ratio of 1:1. The composition of the OBM medium is shown in Table 3 below.

**[Table 3]**

| **OBM medium components** | **Final concentration** |
|---|---|
| A83-01 | 500 nM |
| Y-27632 | 5 mM |
| SB202190 | 500 nM |
| B27 | 1 x |
| N-Acetylcysteine | 1.25 mM |
| Nicotinamide | 5 mM |
| GlutaMax 100x | 1 x |
| HEPES | 10 mM |
| Penicillin/streptomycin | 100 U·ml⁻¹ / 100 mg·ml⁻¹ |
| Advanced DMEM / F12 | 1 x |

On a plate (ultra-low attachment, U-shape plate), 12.5 µl of the above-prepared cancer cells and 12.5 µl of iMSCs were mixed together. Spheroid formation was induced for 1 day in a cell culture incubator at 37°C under 5% CO₂. After the spheroid formation was induced, compaction of the spheroid was induced for additional one day. In this case, when the number of the cells was increased while maintaining the ratio of the cancer cells to the iMSCs at 2:1 (e.g., 1×10³ cells: 5×10² cells), the size of the tumoroid could be increased. However, it was confirmed that the above-mentioned number of cells was most suitable to optimize the additional construction of a fibrotic layer and a vascular cell layer. In addition, the same volume (25 µl) of growth medium-10 was added to induce spheroid compaction. In this case, the maintenance of a serum-free state for more than 24 hours could lead to changes in transcript expression in the cells, and thus a medium containing serum was additionally added. Changes in transcript expression could lead to changes in cell properties, and the final serum concentration was set to 5%. The composition of the growth medium-10 is shown in Table 4 below.

**[Table 4]**

| **Growth medium components** | **Final concentration** |
|---|---|
| Advanced DMEM / F12 | 1 x |
| FBS | 10% |
| Penicillin / streptomycin | 100 U·ml⁻¹ / 100 mg·ml⁻¹ |

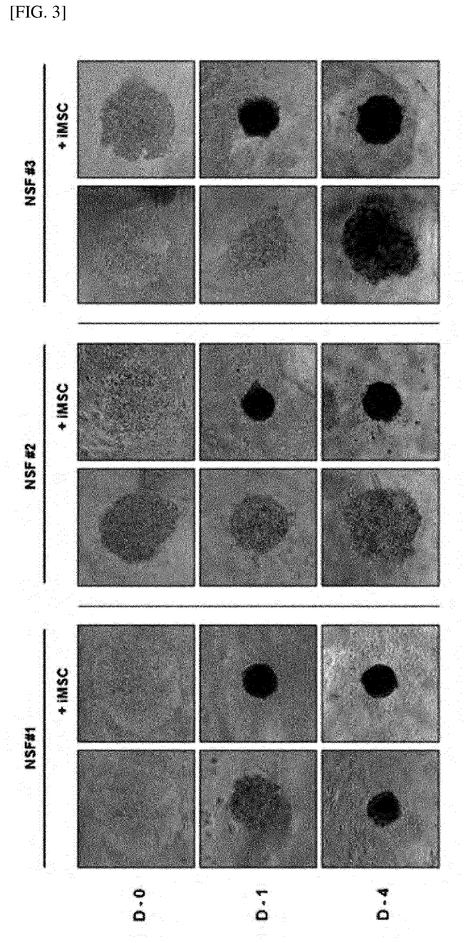

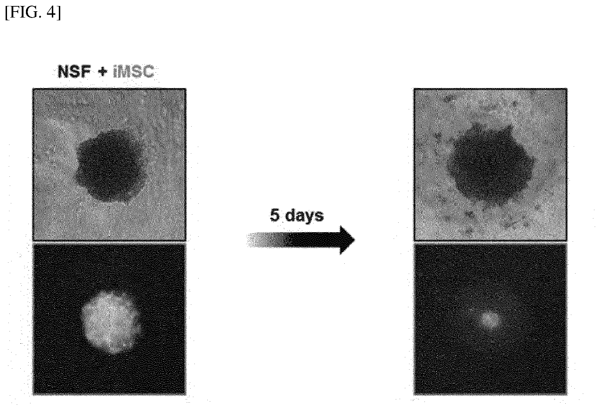

FIG. 3 shows depicts photographs showing the results of inducing spheroid formation from the non-spheroid-forming cells, prepared according to Example 2, in different types of cancer cells. In FIG. 3, NSF#1 represents the result obtained using the breast cancer cell line MDA-MB-231, NSF#2 represents the result obtained using the breast cancer cell line SK-BR-3, and NSF#3 represents the results obtained using the pancreatic cancer cell line AsPC-1. Referring to FIG. 3, it could be confirmed that non-spheroid-forming cells of various carcinomas were formed into spheroids within 1 day by linker cells (iMSCs). As a result of staining the linker cells (which are iMSCs differentiated from iPSCs) with a green fluorescent substance, it was confirmed that 90% or more of the linker cells were dead within 5 days after being used for spheroid formation (see FIG. 4). Due to the death of these linker cells, the characteristics of the tumoroid could be maintained. Another difference from the conventional inventions is that formation of the non-spheroid-forming cells into spheroids was induced using the linker cells.

### Example 3: Induction of fibrotic layer formation (fibrosis-encapsulated tumoroid (FET)) using induced pluripotent stem cells

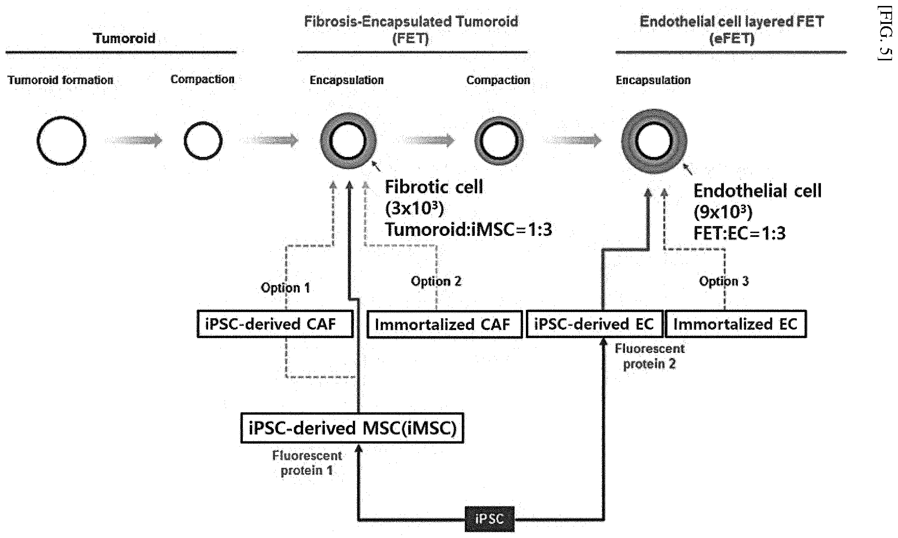

FIG. 5 schematically shows a process of inducing precursor cells (iMSCs) of activated fibroblasts (major cells constituting fibrotic tissue) from induced pluripotent stem cells (iPSCs), producing an FET by inducing the formation of a fibrotic layer on the outside of the tumoroid, and producing an eFET (endothelial cell-layered FET) by inducing the formation of a vascular cell layer. The fibroblast precursor cells derived or originating from induced pluripotent stem cells are called iMSCs, which are differentiated into activated fibroblasts by TGF-β (transforming growth factor-beta) secreted by cancer cells. In this experiment, iMSCs overexpressing a fluorescent protein was used to confirm the formation of the fibrotic layer or the vascular cell layer. First, using encapsulation medium 1, on a plate (ultra-low attachment, U-shape plate), the formation of a fibrotic layer on the outside of the tumoroid was induced using 3×10³ iMSCs that are triple the number of cells used for tumoroid formation. Here, the iMSCs were prepared at a concentration of 1.2×10⁵ cells/ml and used in an amount of 25 µl, and as encapsulation medium 1 used, a mixed medium of equal amounts of TGF-b inhibitor (TI)-free organoid growth medium (OGM) and embryonic body (EB) formation medium (EBFM/AggreWell - STEMCELL Technologies) was used. The composition of the TI-free OGM medium is shown in Table 5 below.

**[Table 5]**

| **TI-free OGM medium components** | **Final concentration** |
|---|---|
| R-Spondin 1 conditioned medium | 10% |
| or R-Spondin 3 | 250 ng·ml⁻¹ |
| Neuregulin 1 | 5 nM |
| FGF 7 | 5 ng·ml⁻¹ |
| FGF 10 | 20 ng·ml⁻¹ |
| EGF | 5 ng·ml⁻¹ |
| Noggin | 100 ng·ml⁻¹ |
| Y-27632 | 5 mM |
| SB202190 | 500 nM |
| B27 supplement | 1x |
| N-acetylcysteine | 1.25 mM |
| Nicotinamide | 5 mM |
| GlutaMax 100x | 1x |
| Hepes | 10 mM |
| Penicillin/streptomycin | 100 U·ml⁻¹ / 100 mg·ml⁻¹ |
| Primocin | 50 mg·ml⁻¹ |
| Advanced DMEM/F12 | 1x |

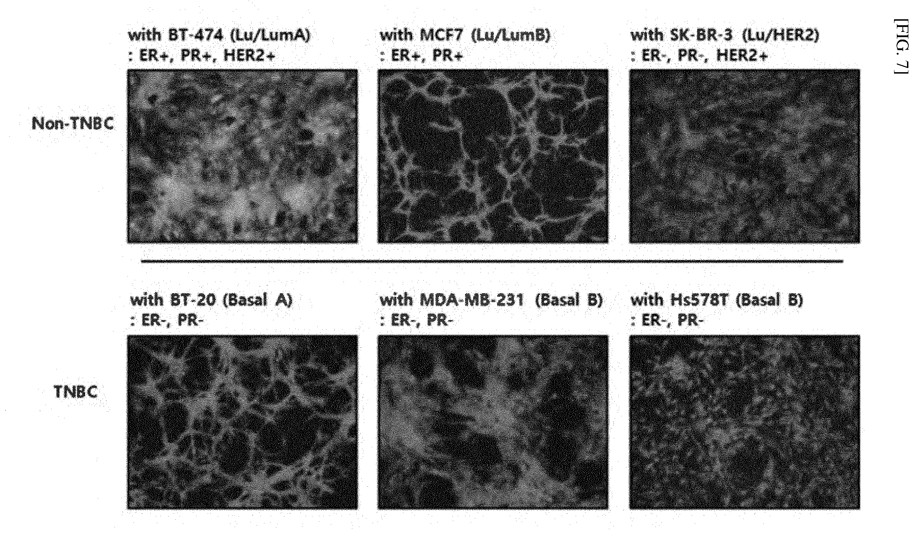

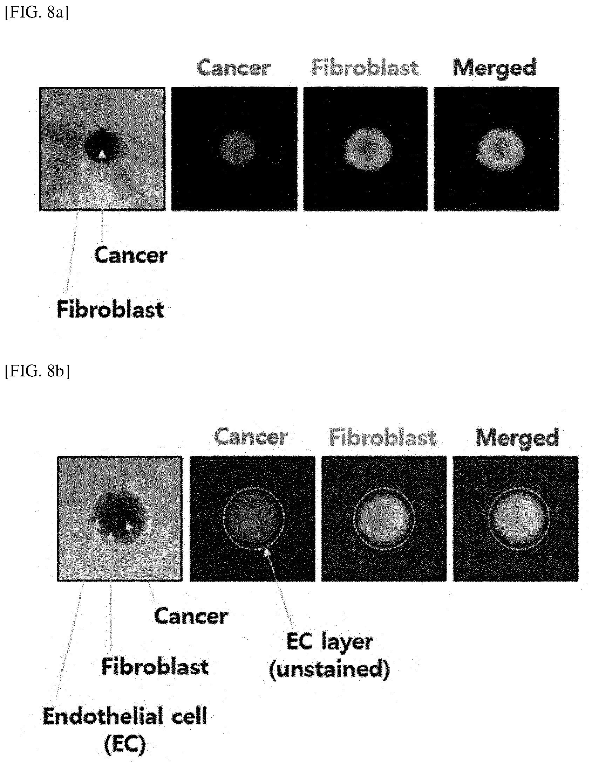

Thereafter, the cells were subjected to spheroid formation and compaction processes in a cell culture incubator at 37°C under 5% CO₂ for additional 2 days. Changes in the morphology of fibroblasts activated by culture media for various human breast cancer cell lines (BT-474, MCF-7, SK-BR-3, BT-20, MDA-MB-231, and Hs578T) that have undergone the above-described processes were observed with a fluorescence microscope, and the results are shown in FIG. 7. In light of the above results, it can be confirmed that various types of fibroblasts were observed depending on the cancer cell line. In addition, unlike a simple tumoroid (Sci Rep. 2016 Jan 11; 6:19103.) consisting of central dead cells, intermediate quiescent cells and outer proliferating cells, a fibrotic layer was formed on the outer layer of a conventional tumoroid, and the resulting tumoroid was fluorescently stained and the fluorescence image thereof was acquired. As a result, as shown in FIG. 8a, it could be confirmed that a fibrotic layer was produced on the outside of the cancer tissue.

### Example 4: Induction of vascular cell layer (endothelial cell-layered FET, eFET) formation using induced pluripotent stem cells

Using encapsulation medium 2, on a plate (ultra-low attachment, U-shape plate), formation of an EC layer on the outside of the FET subjected to the compaction process was induced using 9 × 10³ endothelial cells (ECs) which are triple the number of cells used to form the fibrotic layer. Here, the ECs were prepared at a concentration of 3.6 × 10⁵ cells/ml and used in an amount of 25 µl, and encapsulation medium 2 was prepared by adding 50 ng/ml of VEGFA to encapsulation medium 1. Next, spheroid formation was induced in a cell culture incubator at 37°C under 5% CO₂ for 1 day, and maintenance medium was prepared by adding 20 ng/ml of VEGFA to growth medium-5 shown in Table 6 below. Final spheroid formation was induced by replacing the medium with the maintenance medium.

**[Table 6]**

| **Growth medium 5 components** | **Final concentration** |
|---|---|
| Advanced DMEM / F12 | 1 x |
| FBS | 5% |
| Penicillin / streptomycin | 100 U·ml⁻¹ / 100 mg·ml⁻¹ |

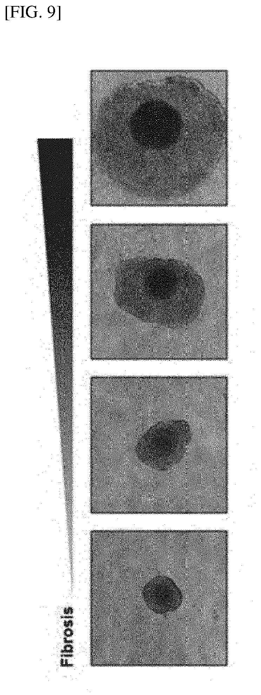

FIG. 8b shows an image of the eFET produced by the above-described method. The vascular endothelial cells were not fluorescently stained, and thus the vascular endothelial cell layer on the fluorescence image was indicated by a white dotted line. It was confirmed that, in the case of the fibrosis-encapsulated tumoroid (FET or eFET) produced by the method of Examples 3 and 4, the thickness of the fibrotic layer varied according to the degree of progression of carcinoma or cancer (see FIG. 9). The thickness of the fibrotic layer can be controlled by controlling the number of fibroblasts, and a fibrosis-encapsulated tumoroid with each fibrotic layer thickness can be produced.

### Example 5: Application of optimal medium composition ratio for efficient EC differentiation

iPSCs were dispensed on a matrigel-coated dish at a density of 4 × 10⁴ cells/cm², and the medium was replaced with pre-warmed N2B27 medium supplemented with CHIR-99021 (8 µM) and hBMP4 (25 ng/ml) in order to induce lateral mesoderm, and the cells were maintained for 3 days without additional medium replacement. The composition of the medium is shown in Table 7 below, and the ratio of DMEM/F12: Meurobasal medium was set to 1:1.

**[Table 7]**

| **N2B27 medium components** | **Final concentration** |
|---|---|
| DMEM/F12 | 1 x |
| Meurobasal medium | 1 x |
| B27 | 1.94% |
| N2 | 0.97% |
| β-mercaptoethanol | 0.097% |

On days 4 and 5, the medium was replaced with StemPro-34 SFM medium supplemented with vascular endothelial growth factor (VEGF, 200 ng/ml) and forskolin (2 µM) in order to induce endothelial cells, and at this time, the medium was replaced daily, unlike during the above three days. On day 6, endothelial cells were sorted and only CD144-positive endothelial cells were selected and plated again. At this time, StemPRo-34 supplemented with VEGF (50 ng/ml) was used as the medium, and the cells were dispensed on fibronectin-coated plates. The composition of the StemPro-34 SFM medium purchased from ThermoFisher Scinentific is shown in Table 8 below.

**[Table 8]**

| **StemPro-34 SFM medium components** | **Final concentration** |
|---|---|
| StemPro-34 SFM medium | 1 x |
| Penicillin / Streptomycin | 100 U·ml⁻¹ / 100 mg·ml⁻¹ |
| GlutaMax 100x | 1 x |

The StemPro-34 medium contains StemPro-34 SFM medium and StemPro-34 supplement. From day 7, the cells were cultured and maintained in Vasculife complete medium.

### Example 6: Examination of anticancer drug resistance of fibrosis-encapsulated tumoroid

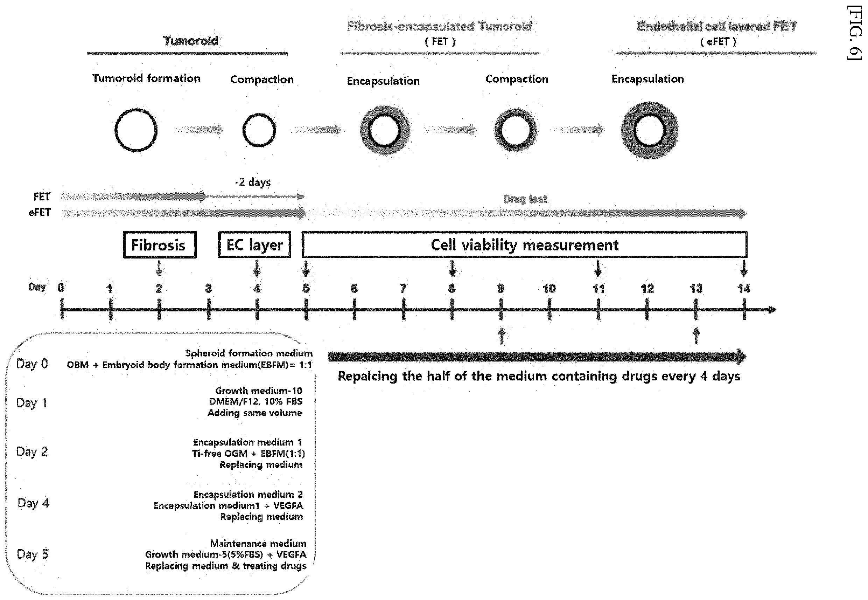

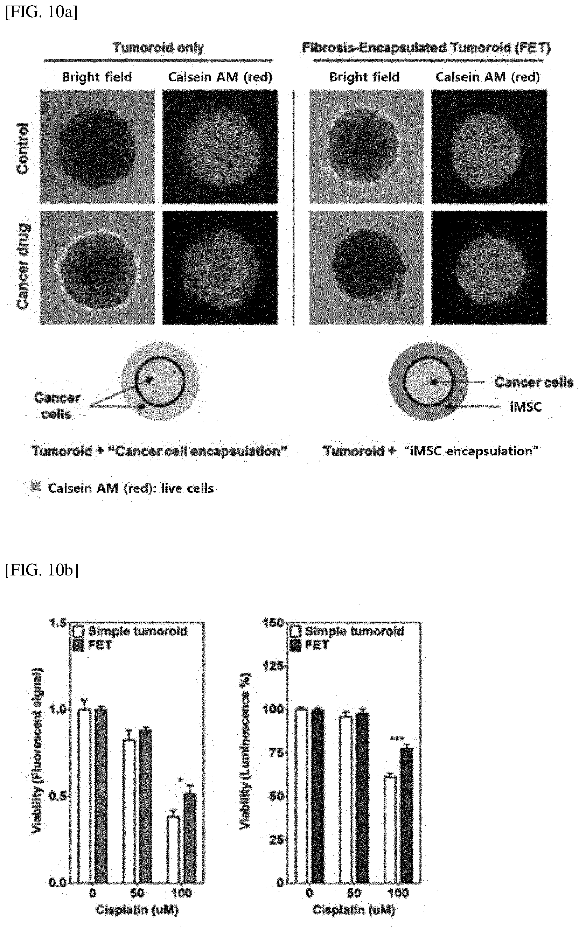

The present inventors performed a drug resistance test in order to evaluate whether it was possible to select drugs, which can be effective in the clinical stage, using the fibrosis-encapsulated tumoroid which is a solid cancer tissue analogue produced by the methods of Examples 1 to 4 (see FIGS. 10a and 10b). In addition, FIG. 6 shows the time flow from the above-described tumoroid formation process to the production of the fibrosis-encapsulated tumoroid and the drug test process. The drug test was conducted through cell viability measurement. The cell viability of each of the group containing the anticancer drug and the control group not containing the anticancer drug was measured after replacing the medium with maintenance medium. At this time, the fluorescent signal was measured using a confocal microscope, and the living cancer cells on the tumeroid that did not display fluorescence were confirmed by staining with Calsein-AM-blue, and the dead cancer cells were confirmed by staining with propidium iodide (PI). The cell viability of the solid cancer tissue analogue was measured using Promega's CellTiter-Glo luminescent cell viability assay as another measurement method, and the death of cancer cells by the anticancer drug could be indirectly confirmed by comparison with the control group. The experimental results are shown in FIGS. 10a and 10b. It was confirmed that the fibrosis-encapsulated tumoroid (FET) showed high anticancer drug resistance, that is, high cell viability, and a stronger Calsein-AM signal, compared to a simple tumoroid (tumor only) (see FIG. 10a). Referring to FIG. 10b, it can be confirmed that the fibrosis-encapsulated tumoroid (FET) shows statistically significant resistance to the anticancer drug (cisplatin, 100 µM), compared to the simple tumoroid. The fibrosis-encapsulated tumoroid produced as described above mimicked a series of processes in which the anticancer agent passes sequentially through the blood vessel wall and fibrotic tissue and reaches cancer tissue, in the same way as in the human body. Thus, it is preferable to finally produce the eFET and conduct an anticancer drug test using the eFET, but only the FET may be selectively used in order to reduce cost or time. When the FET is used, the experimental period can be shortened by 2 days, enabling rapid preemptive selection of anticancer drugs for cancer patients having time and physical limitations. Thus, the FET is expected to be widely used in the precision medical field.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention relates to a method for producing a fibrosis-encapsulated tumoroid (FET) and the use of the fibrosis-encapsulated tumoroid (FET). When the method of the present invention is used, it may dramatically increases the success probability of a candidate substance in the clinical verification stage by perfectly reflecting the characteristics of human solid cancer. Thus, the method may be widely used in the fields of new anticancer drug development and precision medicine.

## Claims

1. A method for producing a fibrosis-encapsulated tumoroid, the method comprising steps of
(a) obtaining cancer cells isolated from a subject; and
(b) co-culturing the cancer cells with induced pluripotent stem cells (iPSCs) or induced pluripotent stem cell-derived cells to form a spheroid-shaped culture.

2. The method of claim 1, wherein the cancer cells comprise non-spheroid-forming cells (NSFs).

3. The method of claim 2, wherein the non-spheroid-forming cells (NSFs) are formed into the spheroid-shaped culture by the induced pluripotent stem cells or iPSC-derived mesenchymal stem cells (iMSCs).

4. The method of claim 1, wherein the co-culture in step (b) is performed by mixing and culturing the cancer cells and the induced pluripotent stem cells (iPSCs) or induced pluripotent stem cell-derived cells at a cell number ratio of 0.01:1 to 100:1.

5. The method of claim 4, wherein the co-culture in step (b) is performed by mixing and culturing the cancer cells and the induced pluripotent stem cells (iPSCs) or induced pluripotent stem cell-derived cells at a cell number ratio of 1.5:1 to 2.5:1.

6. The method of claim 1, further comprising step (c) of inducing a fibrotic layer on the culture.

7. The method of claim 6, further comprising a step of compacting the induced fibrotic layer.

8. The method of claim 6, further comprising step (d) of selecting a fibrosis-encapsulated tumoroid (FET) on which the fibrotic layer has been induced.

9. The method of claim 6, wherein step (c) comprises encapsulating the fibrotic layer by at least one cell type selected from the group consisting of fibroblasts, fibroblast precursor cells, cancer-associated fibroblasts, induced pluripotent stem cell-derived mesenchymal stem cells (iMSCs), and iPSC-derived cancer-associated fibroblasts.

10. The method of claim 8, further comprising step (e) of inducing a vascular cell layer on the fibrotic layer.

11. The method of claim 10, wherein the vascular cell layer is induced by endothelial cells or vascular endothelial cells.

12. The method of claim 11, wherein the endothelial cells or vascular endothelial cells are induced from induced pluripotent stem cells (iPSCs).

13. The method of claim 1, wherein the cancer is a cancer selected from the group consisting of breast cancer, uterine cancer, esophageal cancer, stomach cancer, brain cancer, rectal cancer, colorectal cancer, lung cancer, skin cancer, ovarian cancer, cervical cancer, kidney cancer, blood cancer, pancreatic cancer, prostate cancer, testicular cancer, laryngeal cancer, oral cancer, head and neck cancer, thyroid cancer, liver cancer, bladder cancer, osteosarcoma, lymphoma, leukemia, and combinations thereof.

14. The method of claim 1, wherein the co-culture is performed in at least one culture medium selected from the group consisting of DMEM (Dulbeco's modified Eagle's medium), IMDM (Iscove's modified Dulbecco's medium), a-MEM (alpha modification of Eagle's medium), TI-free OGM (TI-free osteoblast growth medium), F12 (nutrient mixture F-12), RPMI 1640 (RPMI 1640 medium), Williams' s medium E, McCoy's 5A, essential 8 (E8) medium, SFM medium (StemPro-34 SFM medium), N2B2 medium, OBM medium (OGM osteoblast growth medium), growth medium-5, growth medium-10, and DMEM/F12 (Dulbecco's modified Eagle medium: nutrient mixture F-12).

15. The method of claim 14, wherein the culture medium further contains B27, N2, G5, or forskolin.

16. The method of claim 14, wherein the culture medium further contains cancer-associated fibroblasts, endothelial cells, mesenchymal stem cells (iMSCs) differentiated from induced pluripotent stem cells (iPSCs), or a growth factor.

17. The method of claim 16, wherein the growth factor is at least one selected from the group consisting of vascular endothelial growth factor (VEGF), vascular endothelial growth factor A (VEGFA), transforming growth factor beta (TGF-β), basic fibroblast growth factor (bFGF), epidermal growth factor (EGF), and insulin-like growth factor-1 (IGF-1).

18. The method of claim 1, wherein the co-culture is performed for 2 days to 14 days.

19. A fibrosis-encapsulated tumoroid produced by the method of any one of claims 1 to 18.

20. A non-human animal model transplanted with the tumoroid of claim 19.

21. A method for screening a substance for treatment or alleviation of cancer, the method comprising steps of:
(a) preparing the tumoroid of claim 19;
(b) treating the tumoroid with an anticancer candidate substance; and
(c) determining that the candidate substance is the substance for treatment or alleviation of cancer, when the tumoroid exhibits an anticancer effect.
